# EUROPEAN PATENT APPLICATION

(11) **EP 4 193 830 A2**
(43) Date of publication of application: **14.06.2023**
(21) Application number: 22212174.1
(22) Date of filing: 08.12.2022
(51) Int. Cl.: A01H 5/12, A01H 6/14, A01H 1/00

(54) **LETTUCE PLANTS HAVING RESISTANCE TO DOWNY MILDEW**

(30) Priority: 10.12.2021 US 202163288364 P
(71) Applicant: Seminis Vegetable Seeds, Inc., St. Louis MO 63167 (US)
(72) Inventor: HAGEN, Charles, St. Louis, 63167 (US); MORGAN, Robyn L., St. Louis, 63167 (US); SCHRYVE, Philippe, St. Louis, 63167 (US)
(74) Representative: Uexküll & Stolberg

(57) **Abstract**

Lettuce (*Lactuca sativa*) plants exhibiting resistance to downy mildew disease caused by *Bremia lactucae* are provided, together with methods of producing, identifying, or selecting plants or germplasm with a downy mildew resistance phenotype. Such plants include lettuce plants comprising introgressed genomic regions conferring pest resistance. Compositions, including novel polymorphic markers for detecting plants comprising introgressed loci, are further provided.

## Description

### CROSS-REFERENCE TO RELATED APPLICATIONS

This application claims the benefit of U.S. Provisional Appl. Ser. No. 63/288,364, filed December 10, 2021, the disclosure of which is incorporated herein by reference in its entirety.

### INCORPORATION OF SEQUENCE LISTING

A sequence listing containing the file named "SEMB046US_ST26.xml" which is 88.3 kilobytes (measured in MS-Windows^{®}) and created on November 17, 2022, and comprises 85 sequences, is incorporated herein by reference in its entirety.

### FIELD OF THE INVENTION

The present invention relates to methods and compositions for producing lettuce plants exhibiting increased resistance to downy mildew disease caused by *Bremia lactucae.*

### BACKGROUND OF THE INVENTION

Host plant resistance is an important trait in agriculture, particularly in the area of food crop production. Although loci conferring resistance to pests have been identified in various lettuce species, efforts to introduce these loci into cultivated lines have been hindered by a lack of specific markers linked to the loci. The use of marker-assisted selection (MAS) in plant breeding has made it possible to select plants based on genetic markers linked to traits of interest. However, accurate markers for identifying or tracking desirable traits in plants are frequently unavailable even if a gene associated with the trait has been characterized. These difficulties are further complicated by factors such as polygenic or quantitative inheritance, epistasis, and an incomplete understanding of the genetic background underlying expression of a desired phenotype.

### SUMMARY OF THE INVENTION

In one aspect, the present invention provides a *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 2, and wherein said first allele comprises allele 2.2 and wherein said second allele comprises allele 2.1 or *Dm3.* In some embodiments, the recombinant chromosomal segment comprises a marker locus selected from the group consisting of marker locus M1 (SEQ ID NO: 1), marker locus M2 (SEQ ID NO: 6), marker locus M3 (SEQ ID NO: 11), marker locus M4 (SEQ ID NO: 17), marker locus M5 (SEQ ID NO: 21), marker locus M6 (SEQ ID NO: 26), marker locus M7 (SEQ ID NO: 31), and marker locus M8 (SEQ ID NO: 36) on chromosome 2. In other embodiments, said recombinant chromosomal segment comprises marker locus M3 (SEQ ID NO: 11) and a marker locus selected from the group consisting of marker locus M2 (SEQ ID NO: 6), marker locus M5 (SEQ ID NO: 21), marker locus M6 (SEQ ID NO: 26), and marker locus M7 (SEQ ID NO: 31) on chromosome 2. In some embodiments, the plant is homozygous for said recombinant chromosomal segment. In other embodiments, a representative sample of seed comprising said recombinant chromosomal segment has been deposited under NCMA Accession No. 202110051 or NCMA Accession No. 202110049.

In addition, the present invention provides a plant part of a *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 2, and wherein said first allele comprises allele 2.2 and wherein said second allele comprises allele 2.1 or *Dm3,* wherein the plant part comprises the recombinant chromosomal segment. In some embodiments, the plant part is a cell, a seed, a root, a stem, a leaf, a head, a flower, or pollen. In other embodiments, a tissue culture comprising a cell from a plant part of a *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 2, and wherein said first allele comprises allele 2.2 and wherein said second allele comprises allele 2.1 or *Dm3,* wherein the cell comprises the recombinant chromosomal segment.

The present invention provides a recombinant DNA segment comprising a first *Bremia lactucae* resistance allele and a second *Bremia lactucae* resistance allele, wherein said first allele and second allele are in *cis* configuration, and wherein said first allele comprises allele 2.2 and wherein said second allele comprises allele 2.1 or *Dm3.* In some embodiments, said recombinant DNA segment comprises the sequence of marker locus M3 (SEQ ID NO: 11) and a sequence selected from the group consisting of marker locus M2 (SEQ ID NO: 6), marker locus M5 (SEQ ID NO: 21), and marker locus M6 (SEQ ID NO: 26). In other embodiments, said recombinant DNA segment is further defined as comprised within a plant, plant part, plant cell, or seed. In further embodiments, a representative sample of seed comprising said DNA segment has been deposited under NCMA Accession No. 202110051 or NCMA Accession No. 202110049.

In antoher aspect, the present invention provides a *Lactuca sativa* plant comprising a recombinant chromosomal segment that comprises a first allele that confers resistance to *Bremia lactucae* and a second allele that confers resistance to *Bremia lactucae* wherein said first allele and second allele are in *cis* configuration on chromosome 4, and wherein said first allele comprises allele 4.1 and wherein said second allele comprises allele 4.2 or allele 4.3. In some embodiments, said recombinant chromosomal segment comprises a marker selected from the group consisting of marker locus M9 (SEQ ID NO: 41), marker locus M10 (SEQ ID NO: 46), marker locus M11 (SEQ ID NO: 51), marker locus M12 (SEQ ID NO: 56), marker locus M13 (SEQ ID NO: 61), marker locus M14 (SEQ ID NO:66), marker locus M15 (SEQ ID NO: 71), marker locus M16 (SEQ ID NO: 76), and marker locus M17 (SEQ ID NO: 81) on chromosome 4. In other embodiments, said recombinant chromosomal segment comprises marker locus selected from the group consisting of marker locus M9 (SEQ ID NO: 41), marker locus M10 (SEQ ID NO: 46), marker locus M11 (SEQ ID NO: 51), marker locus M16 (SEQ ID NO: 76), and marker locus M17 (SEQ ID NO: 81) and a marker locus selected from the group consisting of marker locus M12 (SEQ ID NO: 56), marker locus M13 (SEQ ID NO: 61, marker locus M14 (SEQ ID NO: 66), and marker locus M15 (SEQ ID NO: 71) on chromosome 4. In some embodiments, the plant is homozygous for said recombinant chromosomal segment. In other embodiments, a representative sample of seed comprising said recombinant chromosomal segment has been deposited under NCMA Accession No. 202110050 or NCMA Accession No. 202110052. Seed that produce the lettuce plants of the present invention are also provided herein.

In addition, the present invention provides a plant part of a *Lactuca sativa* plant comprising a recombinant chromosomal segment that comprises a first allele that confers resistance to *Bremia lactucae* and a second allele that confers resistance to *Bremia lactucae* wherein said first allele and second allele are in *cis* configuration on chromosome 4, and wherein said first allele comprises allele 4.1 and wherein said second allele comprises allele 4.2 or allele 4.3, wherein the plant part comprises the recombinant chromosomal segment. In some embodiments, the plant part is a cell, a seed, a root, a stem, a leaf, a head, a flower, or pollen. In other embodiments, a tissue culture comprising a cell from a plant part of a *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 4, and wherein said first allele comprises allele 4.1 and wherein said second allele comprises allele 4.2 or allele 4.3, wherein the cell comprises the recombinant chromosomal segment.

The present invention provides a recombinant DNA segment comprising a first *Bremia lactucae* resistance allele and a second *Bremia lactucae* resistance allele, wherein said first allele and second allele are in *cis* configuration, and wherein said first allele comprises allele 4.1 and wherein said second allele comprises allele 4.2 or allele 4.3. In some embodiments, said recombinant DNA segment comprises the sequence of marker locus M9 (SEQ ID NO: 41), marker locus M10 (SEQ ID NO: 46), marker locus M11 (SEQ ID NO: 51), marker locus M16 (SEQ ID NO: 76), and marker locus M17 (SEQ ID NO: 81) and a marker locus selected from the group consisting of marker locus M12 (SEQ ID NO: 56), marker locus M13 (SEQ ID NO: 61, marker locus M14 (SEQ ID NO: 66), and marker locus M15 (SEQ ID NO: 71). In other embodiments, said recombinant DNA segment is further defined as comprised within a plant, plant part, plant cell, or seed. In further embodiments, a representative sample of seed comprising said chromosomal segment has been deposited under NCMA Accession No. 202110050 or NCMA Accession No. 202110052.

In another aspect, the present invention provides a method for producing a *Lactuca sativa* plant with broad-spectrum resistance to *Bremia lactucae* comprising introgressing into said plant a recombinant chromosomal segment comprising a first *Bremia lactucae* resistance allele and a second *Bremia lactucae* resistance allele within a recombinant chromosomal segment flanked in the genome of said plant by: (a) marker locus M1 (SEQ ID NO: 1) and marker locus M4 (SEQ ID NO: 17) on chromosome 2; or (b) marker locus M9 (SEQ ID NO: 41) and marker locus M17 (SEQ ID NO: 81) on chromosome 4, wherein said first and second *Bremia lactucae* resistance alleles confer to said plant broad-spectrum resistance to *Bremia lactucae* relative to a plant lacking said alleles, and wherein said introgressing comprises marker-assisted selection. In some embodiments, said introgressing comprises: a) crossing a plant comprising said recombinant chromosomal segment with itself or with a second *Lactuca sativa* plant of a different genotype to produce one or more progeny plants; and b) selecting a progeny plant comprising said recombinant chromosomal segment. In other embodiments, selecting a progeny plant comprises detecting nucleic acids comprising: (a) marker locus M1 (SEQ ID NO: 1), marker locus M2 (SEQ ID NO: 6), marker locus M3 (SEQ ID NO: 11), marker locus M4 (SEQ ID NO: 17), marker locus M5 (SEQ ID NO: 21), marker locus M6 (SEQ ID NO: 26), marker locus M7 (SEQ ID NO: 31), or marker locus M8 (SEQ ID NO: 36); or (b) marker locus M9 (SEQ ID NO: 41), marker locus M10 (SEQ ID NO: 46), marker locus M11 (SEQ ID NO: 51), marker locus M12 (SEQ ID NO: 56), marker locus M13 (SEQ ID NO: 61), marker locus M14 (SEQ ID NO: 66), marker locus M15 (SEQ ID NO: 71), marker locus M16 (SEQ ID NO: 76), or marker locus M17 (SEQ ID NO: 81). In further embodiments, the progeny plant is an F₂-F₆ progeny plant. In some embodiments, said introgressing further comprises backcrossing or assaying for said resistance to *Bremia lactucae.* The present invention further provides lettuce plants obtainable by the methods provided herein.

The present invention provides a method of selecting a *Lactuca sativa* plant exhibiting resistance to *Bremia lactucae,* comprising: a) crossing a *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 2, and wherein said first allele comprises allele 2.2 and wherein said second allele comprises allele 2.1 or *Dm3* or a *Lactuca sativa* plant comprising a recombinant chromosomal segment that comprises a first allele that confers resistance to *Bremia lactucae* and a second allele that confers resistance to *Bremia lactucae* wherein said first allele and second allele are in *cis* configuration on chromosome 4, and wherein said first allele comprises allele 4.1 and wherein said second allele comprises allele 4.2 or allele 4.3, with itself or with a second *Lactuca sativa* plant of a different genotype to produce one or more progeny plants; and b) selecting a progeny plant comprising said recombinant chromosomal segment. In some embodiments, selecting said progeny plant detecting a marker locus genetically linked to said recombinant chromosomal segment. In other embodiments, selecting said progeny plant comprises detecting a marker locus within or genetically linked to a chromosomal segment flanked in the genome of said plant by: (a) marker locus M1 (SEQ ID NO: 1) and marker locus M4 (SEQ ID NO: 16) on chromosome 2; or (b) marker locus M9 (SEQ ID NO: 41) and marker locus M17 (SEQ ID NO:_81) on chromosome 4. In some embodiments, selecting a progeny comprises detecting nucleic acids comprising: (a) marker locus M1 (SEQ ID NO: 1), marker locus M2 (SEQ ID NO: 6), marker locus M3 (SEQ ID NO: 11), marker locus M4 (SEQ ID NO: 17), marker locus M5 (SEQ ID NO: 21), marker locus M6 (SEQ ID NO: 26), marker locus M7 (SEQ ID NO: 31), or marker locus M8 (SEQ ID NO: 36); or (b) marker locus M9 (SEQ ID NO: 41), marker locus M10 (SEQ ID NO: 46), marker locus M11 (SEQ ID NO: 51), marker locus M12 (SEQ ID NO: 56), marker locus M13 (SEQ ID NO: 61), marker locus M14 (SEQ ID NO: 66), marker locus M15 (SEQ ID NO: 71), marker locus M16 (SEQ ID NO: 76), or marker locus M17 (SEQ ID NO: 81). In other embodiments, producing said progeny plant comprises backcrossing. In further embodiments, said progeny plant is an F₂-F₆ progeny plant. The present invention further provides lettuce plants produced by the methods provided herein.

The *Lactuca sativa* plants of the present invention preferably are elite *Lactuca sativa* plants.

### BRIEF DESCRIPTION OF THE DRAWINGS

FIG. 1 shows an overview of fine-mapping results for *B. lactucae* resistance alleles of the gene cluster on chromosome 2. The black bar indicates which genomic intervals are associated with *B. lactucae* resistance for that particular allele.
FIG. 2 shows an overview of fine-mapping results for *B. lactucae* resistance alleles of the gene cluster on chromosome 4. The dark grey bar indicates which genomic intervals are associated with *B. lactucae* resistance for that particular allele.
FIG. 3 shows an overview of four cis-linked events on chromosome 2 (left graph) and on chromosome 4 (right graph).

### DETAILED DESCRIPTION

Lettuce (*L*. *sativa*) is an important vegetable crop that is produced and consumed on a global scale. The most important disease that affects the quality of lettuce crops is downy mildew, caused by the oomycete *Bremia lactucae.* To control *B. lactucae* infection in lettuce, breeders typically employ a strategy that combines the use of fungicides with lettuce varieties that have genetic resistance to *B. lactucae.* However, fungicide use is expensive and heavy use can lead to the development of *B. lactucae* races that are no longer susceptible to fungicides. In addition, increasingly restrictive regulations over fungicide use along with a consumer push for reduced pesticide use limits the options for *B. lactucae* control measures by lettuce growers. These factors have led growers to rely more on the genetic resistance of the lettuce varieties they grow. However, pathogen evolution has reduced the efficacy of the resistance genes typically deployed to protect lettuce crops. Thirty-six different *B. lactucae* races have been identified to date but novel races are discovered every year. These races are evaluated and officially named by the International Bremia Evaluation Board (IBEB). It is a priority for lettuce breeders to develop lettuce varieties having resistance to all commercially relevant known *B. lactucae* races, and for breeders of new lettuce varieties, it is desirable to develop lettuce varieties that are resistant to *B. lactucae* races Bl to B16 and other additional relevant races.

More than 50 genes that confer resistance to *B. lactucae* infection have been identified in *L. sativa* and its wild relatives *L. serriola* and *L. saligna.* Breeders have been able to utilize the genes from *L*. *serriola* and *L*. *saligna* in the production of resistant lettuce varieties due to the fact that *L. serriola* and *L. saligna* are closely related to *L. sativa.* Several *B. lactucae* resistance genes have also been identified in the wild relative *L. virosa,* but introgression of genes from *L. virosa* into *L. sativa* is challenging due to lingering fertility barriers. The known resistance genes from *L*. *serriola* can be mapped to two gene clusters in the lettuce genome. One cluster is located on chromosome 2 while the other cluster is located on chromosome 4. While breeders appear to have multiple resistance genes to choose from, the location of the genes in such gene clusters and the proximity between the genes has greatly limited the ability to stack available resistance genes.

After a new *B. lactucae* isolate is determined to be commercially relevant, a lettuce breeder must identify a resistance gene that confers resistance to the new isolate and incorporate this additional resistance locus along with the loci conferring resistance to all previously known isolates into a relevant lettuce variety. One method used to identify genes that confer resistance to new *B. lactucae* isolates is through screening of wild lettuce accessions. Although this method has resulted in the identification ofa large number of resistance alleles, it is unpredictable. Another method to identify genes that confer resistance to new *B. lactucae* isolates is to evaluate known resistance genes to determine if the known gene confers resistance to one or more of the new isolate(s). After a source of resistance to the new isolate(s) is identified, the resistance locus must be incorporated into the relevant lettuce germplasm for use in the breeding program to develop new lettuce varieties. If a known gene confers resistance to the new isolate, it is easy for the breeders to incorporate the resistance locus into the breeding program. However, if a new resistance allele is required to confer resistance, the new allele must be integrated into varieties that likely already include other loci that confer resistance to other *B*. *lactucae* races. Such new allele must be introgressed along with any other relevant loci so as to ensure that the resulting plants or varieties exhibit resistance to all known commercially relevant B. lactucae races or isolates. As an alternative strategy, there have been attempts to develop resistance alleles that confer broad-spectrum resistance to *B. lactucae,* specifically resistance to all known commercially relevant *B. lactucae* races. However, to date, no broad-spectrum resistance alleles have been successfully identified or used. Although multiple *B. lactucae* resistance genes have been combined in a single lettuce variety, , the combination of these genes did not result in broad spectrum resistance or a resistance profile that provided resistance to all known commercially relevant *B. lactucae* races. The development of new resistance alleles that confer broad-spectrum resistance to *B. lactucae* from existing resistance genes residing in the same gene cluster is nearly impossible without additional information regarding the resistance genes and genetic markers for said genes.

The invention represents a significant advance in the art by providing *L. sativa* plants having a novel resistance allele that comprises multiple *B. lactucae* resistance loci in a cis-configuration at the chromosome 2 gene cluster and *L. sativa* plants having a novel resistance allele comprising multiple *B. lactucae* resistance loci in a cis-configuration at the chromosome 4 gene cluster. Each novel allele can be introgressed into lettuce varieties to confer broad-spectrum resistance to *B. lactucae* to plants of such varieties. Such plants can be referred to as plants of *B. lactucae* resistant lettuce varieties. Methods of producing such *B. lactucae* resistant lettuce plants, lines, and varieties are further provided. Also disclosed herein are molecular markers that are linked to quantitative trait loci (QTL) contributing to *B. lactucae* resistance. Through use of such markers and the methods described herein, one of skill in the art may increase the level of *B. lactucae* resistance exhibited by a lettuce plant and identify plants exhibiting an increased level of *B. lactucae* resistance.

Previously, all *B. lactucae* resistance loci assigned to either the chromosome 2 or chromosome 4 were believed to be allelic to the loci within each cluster. After a resistance locus had been sorted into one of the known gene clusters, further fine-mapping of the locus was not pursued as the locus was considered to be allelic with the other loci assigned to the gene cluster. However, it was shown that the resistance loci are not allelic but are in fact different genes that have resistance patterns against different races of *B. lactucae* and that three resistance genes could be placed in a cis-configuration at the same gene cluster to produce a novel *B. lactucae* resistance allele (WO 2013124310 A1). However, the allele described in WO 2013124310 only conferred resistance to a subset of *B. lactucae* races. Although WO 2013124310 dispelled the belief that the *B. lactucae* resistance loci at the gene clusters are allelic, it still remains difficult to obtain an allele conferring broad-spectrum resistance to *B. lactucae* through recombination due to the narrow genomic region encompassed by each of the gene clusters. The process of combining genes to generate an allele conferring broad-spectrum resistance to *B. lactucae* also carries the potential of inadvertently losing resistance to one or more *B. lactucae* races or isolate because the relevant resistance genes are not present in the resulting variety. In addition, different alleles might include resistance genes having different resistance profiles located in the same location on the chromosome limiting the combination on a single chromosome.

The present invention represents a significant advance in that it provides, in one embodiment, a novel allele on chromosome 2 which can be introgressed in a lettuce plant to conferbroad spectrum resistance to *B. lactucae,* as well as methods for the production thereof. In another embodiment, the present invention provides a novel allele on chromosome 4 which can be introgressed in a lettuce plant to confer broad-spectrum resistance to *B. lactucae,* as well as methods for the production thereof. It was surprisingly found that multiple genes within each of the gene clusters could be recombined into a cis-configuration on the chromosome to produce a novel resistance allele or coupling event and that the allele provides resistance to all known commercially relevant *B. lactucae* races. Novel markers for the new alleles are provided herein, allowing the alleles to be accurately introgressed and tracked during development of new varieties. The novel *B. lactucae* resistance alleles can be introgressed into any desired lettuce variety.

The present invention provides a recombinant chromosomal segment on chromosome 2 comprising a novel *B. lactucae* resistance allele and lettuce plants comprising such recombinant chromosomal segment. Surprisingly, this *B. lactucae* resistance allele provides resistance to all known commercially relevant *B. lactucae* races. Methods of producing such plants comprising the resistance are further provided. In some embodiments, the novel *B. lactucae* resistance allele is defined as located within a recombinant chromosomal segment flanked by marker locus M1 (SEQ ID NO: 1) and marker locus M4 (SEQ ID NO: 16) on chromosome 2. In other embodiments, such a segment can comprise one or more of marker locus M2 (SEQ ID NO: 6) and marker locus M3 (SEQ ID NO: 11). Marker locus M1 comprises a SNP change from C to T at 3,178,102 bp of the public *L. sativa* reference genome Lsat_Salinas_v8, marker locus M4 comprises a SNP change from C to T at 21,382,669 bp of the public *L. sativa* reference genome Lsat_Salinas_v8, marker locus M2 comprises a SNP change from A to T at 4,751,330 bp of the public *L*. *sativa* reference genome Lsat_Salinas_v8, and marker locus M3 comprises a SNP change from C to T at 12,617,612 bp of the public *L. sativa* reference genome Lsat_Salinas_v8. The public genome of lettuce is available at, for example lgr.genomecenter.ucdavis.edu, and one skilled in the art would understand how to locate the marker sequences provided for the first time in the instant application on any version (or later version) of the public genome.

In other embodiments, the novel *B. lactucae* resistance allele is defined as located within a recombinant chromosomal segment flanked by marker locus M5 (SEQ ID NO: 21) and marker locus M8 (SEQ ID NO: 36) on chromosome 2. In other embodiments, such a segment can comprise one or more of marker locus M3 (SEQ ID NO: 11), marker locus M6 (SEQ ID NO: 26), and marker locus M7 (SEQ ID NO: 31). Marker locus M5 comprises a SNP change from A to T at 6,880,789 bp of the public *L. sativa* reference genome Lsat_Salinas_v8, marker locus M8 comprises a SNP change from T to C at 12,620,486 bp of the public *L. sativa* reference genome Lsat_Salinas_v8, marker locus M3 comprises a SNP change from C to T at 12,617,612 bp of the public *L. sativa* reference genome Lsat_Salinas_v8, marker locus M6 comprises a SNP change from A to G at 8,595,550 bp of the public *L. sativa* reference genome Lsat_Salinas_v8, and marker locus M7 comprises a SNP change from C to T at 9,015,255 bp of the public *L. sativa* reference genome Lsat_Salinas_v8. The public genome of lettuce is available at, for example lgr.genomecenter.ucdavis.edu, and one skilled in the art would understand how to locate the marker sequences provided for the first time in the instant application on any version (or later version) of the public genome.

The present invention provides a recombinant chromosomal segment on chromosome 4 comprising a novel *B. lactucae* resistance allele and lettuce plants comprising such recombinant chromosomal segment. Surprisingly, this *B. lactucae* resistance allele provides resistance to all known commercially relevant *B. lactucae* races. Methods of producing such plants comprising the resistance are further provided. In some embodiments, the novel *B. lactucae* resistance allele is defined as located within a recombinant chromosomal segment flanked by marker locus M9 (SEQ ID NO: 41) and marker locus M13 (SEQ ID NO: 61) on chromosome 4. In other embodiments, such a segment can comprise one or more of marker locus M10 (SEQ ID NO: 46), marker locus M11 (SEQ ID NO: 51), and marker locus M12 (SEQ ID NO: 56). Marker locus M9 comprises a SNP change from A to T at 279,265,815 bp of the public *L. sativa* reference genome Lsat_Salinas_v8, marker locus M13 comprises a SNP change from C to T at 299,871,312 bp of the public *L. sativa* reference genome Lsat_Salinas_v8, marker locus M10 comprises a SNP change from A to T at 284,924,768 bp of the public *L. sativa* reference genome Lsat_Salinas_v8, marker locus M11 comprises a SNP change from C to T at 285,707,267 bp of the public *L. sativa* reference genome Lsat_Salinas_v8, and marker locus M12 comprises a SNP change from G to A at 287,073,248 bp of the public *L. sativa* reference genome Lsat_Salinas_v8. The public genome of lettuce is available at, for example lgr.genomecenter.ucdavis.edu, and one skilled in the art would understand how to locate the marker sequences provided for the first time in the instant application on any version (or later version) of the public genome.

In other embodiments, the novel *B. lactucae* resistance allele is defined as located within a recombinant chromosomal segment flanked by marker locus M14 (SEQ ID NO: 66) and marker locus M17 (SEQ ID NO: 81) on chromosome 4. In other embodiments, such a segment can comprise one or more of marker locus M15 (SEQ ID NO:71) and marker locus M16 (SEQ ID NO:76). Marker locus M14 comprises a SNP change from T to C at 272,188,909 bp of the public *L. sativa* reference genome Lsat_Salinas_v8, marker locus M17 comprises a SNP change from C to T at 286,940,580 bp of the public *L. sativa* reference genome Lsat_Salinas_v8, marker locus M15 comprises a SNP change from A to G at 284,076,880 bp of the public *L. sativa* reference genome Lsat_Salinas_v8 and marker locus M16 comprises a SNP change from T to C at 285,706,267 bp of the public *L. sativa* reference genome Lsat_Salinas_v8. The public genome of lettuce is available at, for example lgr.genomecenter.ucdavis.edu, and one skilled in the art would understand how to locate the marker sequences provided for the first time in the instant application on any version (or later version) of the public genome.

In certain embodiments, the invention provides methods of producing or selecting a lettuce plant exhibiting resistance to *B. lactucae* comprising: a) crossing a lettuce plant provided herein with itself or with a second lettuce plant of a different genotype to produce one or more progeny plants; and b) selecting a progeny plant comprising a *B. lactucae* resistance allele. In some embodiments, methods of the invention comprise selecting a progeny plant by detecting nucleic acids comprising marker locus M2 (SEQ ID NO: 6), marker locus M3 (SEQ ID NO: 11), marker locus M5 (SEQ ID NO: 21), marker locus M6 (SEQ ID NO: 26), marker locus M7 (SEQ ID NO: 31), marker locus M10 (SEQ ID NO: 46), marker locus M11 (SEQ ID NO: 51), marker locus M12 (SEQ ID NO: 56), marker locus M15 (SEQ ID NO: 71), or marker locus M16 (SEQ ID NO: 76).

Because genetically diverse plant lines can be difficult to cross, the introgression of *B. lactucae* resistance loci and/or alleles into cultivated lines using conventional breeding methods could require prohibitively large segregating populations for progeny screens with an uncertain outcome. Marker-assisted selection (MAS) is therefore essential for the effective introgression of loci that confer resistance *B. lactucae* into cultivars. For the first time, the present invention enables effective MAS by providing improved and validated markers for detecting genotypes associated with *B. lactucae* resistance without the need to grow large populations of plants to maturity in order to observe the phenotype.

### I. Genomic Regions, Loci, and Polymorphisms in Lettuce Associated With Resistance to Downy Mildew Disease

The invention provides novel introgressions of one or more loci associated with resistance to downy mildew disease in lettuce, together with polymorphic nucleic acids and linked markers for tracking the introgressions during plant breeding.

*Lactuca sativa* accessions exhibiting *B. lactucae* resistance are known in the art and may be used in accordance with certain embodiments of the invention. It may also be possible to use other lettuce types including *L. serriola, L. virosa,* and *L*. *saligna. Lactuca sativa* accession CGN05813 and *Lactuca saligna* accession CGN05271, which can be obtained from the Center for Genetic Resources (Wageningen, The Netherlands), also exhibit resistance to *B. lactucae.* Accessions for *L*. *serriola* lines exhibiting resistance to various *B. lactucae* races are given, for example, in Lebeda et al., Eur J Plant Pathol, 138:597-640, 2014. Other *B. lactucae* resistance sources have also been described and are known in the art (see, for example, Simko et al., Phytopathology 105(9):1220-1228, 2015; Van Hese et al., Eur J. Plant Pathology 144:431-441, 2016; and Parra et al., Euphytica 210:309-326, 2016). *Lactuca* accessions have been collected in numerous locales including France and Portugal and can be found in a number of germplasm banks including Center for Genetic Resources, the Netherlands (CGN) and National Plant Germplasm System (NPGS).

### II. Introgression of Genomic Regions Associated with Resistance to Downy Mildew Disease

Marker-assisted introgression involves the transfer of a chromosomal region defined by one or more markers from a first genetic background to a second. Offspring of a cross that contain the introgressed genomic region can be identified by the combination of markers characteristic of the desired introgressed genomic region from a first genetic background and both linked and unlinked markers characteristic of the second genetic background.

The present invention provides novel accurate markers for identifying and tracking introgression of one or more of the genomic regions disclosed herein from a downy mildew resistant plant into a cultivated line. The invention further provides markers for identifying and tracking the novel introgressions disclosed herein during plant breeding, including the markers set forth in Tables 1 and 2.

Markers within or linked to any of the genomic intervals of the present invention may be useful in a variety of breeding efforts that include introgression of genomic regions associated with pest resistance into a desired genetic background. For example, a marker within 40 cM, 20 cM, 15 cM, 10 cM, 5cM, 2 cM, or 1 cM of a marker associated with pest resistance described herein can be used for marker-assisted introgression of genomic regions associated with a pest resistant phenotype.

Lettuce plants comprising one or more introgressed regions associated with a desired phenotype wherein at least 10%, 25%, 50%, 75%, 90%, or 99% of the remaining genomic sequences carry markers characteristic of the recurrent parent germplasm are also provided. Lettuce plants comprising an introgressed region comprising regions closely linked to or adjacent to the genomic regions and markers provided herein and associated with a pest resistance phenotype are also provided.

### III. Development of Lettuce Varieties Resistant to Downy Mildew Disease

For most breeding objectives, commercial breeders work with germplasm that is "cultivated," "cultivated type," or "elite". This germplasm is easier to breed because it generally performs well when evaluated for horticultural performance. A number of cultivated lettuce types have been developed, including *L. sativa,* which is agronomically elite and appropriate for commercial cultivation. Lettuce cultivar groups include, but are not limited to, the Cos, Cutting, Stalk (or Asparagus), Butterhead, Crisphead (or Iceberg or Cabbage), Latin and Oilseed groups (De Vries, Gen. Resources and Crop Evol. 44:165-174, 1997). However, the performance advantage a cultivated germplasm provides can be offset by a lack of allelic diversity. Breeders generally accept this tradeoff because progress is faster when working with cultivated material than when breeding with genetically diverse sources.

In contrast, when cultivated germplasm is crossed with non-cultivated germplasm, a breeder can gain access to novel alleles from the non-cultivated type. However, this approach presents significant difficulties due to fertility problems associated with crosses between diverse lines, and negative linkage drag from the non-cultivated parent. In lettuce plants, non-cultivated types such as *L*. *serriola* can provide alleles associated with disease resistance. However, these non-cultivated types may have poor horticultural qualities.

The process of introgressing desirable resistance genes from non-cultivated lines into elite cultivated lines while avoiding problems with genetically linked deleterious loci or low heritability is a long and often arduous process. In deploying loci derived from wild relatives it is often desirable to introduce a minimal or truncated introgression that provides the desired trait but lacks detrimental effects. To aid introgression reliable marker assays are preferable to phenotypic screens. Success is furthered by simplifying genetics for key attributes to allow focus on genetic gain for quantitative traits such as pest resistance. Moreover, the process of introgressing genomic regions from non-cultivated lines can be greatly facilitated by the availability of accurate markers for MAS.

One of skill in the art would therefore understand that the loci, polymorphisms, and markers provided by the invention allow the tracking and introduction of any of the genomic regions identified herein into any genetic background. In addition, the genomic regions associated with pest resistance disclosed herein can be introgressed from one genotype to another and tracked using MAS. Thus, the inventors' discovery of accurate markers associated with pest resistance will facilitate the development of lettuce plants having beneficial phenotypes. For example, seed can be genotyped using the markers of the present invention to select for plants comprising desired genomic regions associated with pest resistance. Moreover, MAS allows identification of plants homozygous or heterozygous for a desired introgression.

Inter-species crosses can also result in suppressed recombination and plants with low fertility or fecundity. For example, suppressed recombination has been observed for the tomato nematode resistance gene *Mi,* the *Mla* and *Mlg* genes in barley, the *Yr17* and *Lr20* genes in wheat, the *Run1* gene in grapevine, and the *Rma* gene in peanut. Meiotic recombination is essential for classical breeding because it enables the transfer of favorable loci across genetic backgrounds, the removal of deleterious genomic fragments, and pyramiding traits that are genetically tightly linked. Therefore, suppressed recombination forces breeders to enlarge segregating populations for progeny screens in order to arrive at the desired genetic combination.

Phenotypic evaluation of large populations is time-consuming, resource-intensive and not reproducible in every environment. Marker-assisted selection offers a feasible alternative. Molecular assays designed to detect unique polymorphisms, such as SNPs, are versatile. However, they may fail to discriminate loci within and among lettuce species in a single assay. Structural rearrangements of chromosomes such as deletions impair hybridization and extension of synthetically labeled oligonucleotides. In the case of duplication events, multiple copies are amplified in a single reaction without distinction. The development and validation of accurate and highly predictive markers are therefore essential for successful MAS breeding programs.

### IV. Marker Assisted Breeding and Genetic Engineering Techniques

Genetic markers that can be used in the practice of the present invention include, but are not limited to, restriction fragment length polymorphisms (RFLPs), amplified fragment length polymorphisms (AFLPs), simple sequence repeats (SSRs), simple sequence length polymorphisms (SSLPs), single nucleotide polymorphisms (SNPs), insertion/deletion polymorphisms (Indels), variable number tandem repeats (VNTRs), and random amplified polymorphic DNA (RAPD), isozymes, and other markers known to those skilled in the art. Marker discovery and development in crop plants provides the initial framework for applications to marker-assisted breeding activities (U.S. Patent Pub. Nos.: 2005/0204780, 2005/0216545, 2005/0218305, and 2006/00504538). The resulting "genetic map" is the representation of the relative position of characterized loci (polymorphic nucleic acid markers or any other locus for which loci can be identified) to each other.

Polymorphisms comprising as little as a single nucleotide change can be assayed in a number of ways. For example, detection can be made by electrophoretic techniques including a single strand conformational polymorphism (Orita et al. (1989) Genomics, 8(2), 271-278), denaturing gradient gel electrophoresis (Myers (1985) EP 0273085), or cleavage fragment length polymorphisms (Life Technologies, Inc., Gaithersburg, MD), but the widespread availability of DNA sequencing often makes it easier to simply sequence amplified products directly. Once the polymorphic sequence difference is known, rapid assays can be designed for progeny testing, typically involving some version of PCR amplification of specific loci (PASA; Sommer et al. (1992) Biotechniques 12(1), 82-87), or PCR amplification of multiple specific loci (PAMSA; Dutton and Sommer (1991) Biotechniques, 11(6), 700-7002).

Polymorphic markers serve as useful tools for assaying plants for determining the degree of identity of lines or varieties (U.S. Patent No. 6,207,367). These markers form the basis for determining associations with phenotypes and can be used to drive genetic gain. In certain embodiments of methods of the invention, polymorphic nucleic acids can be used to detect in a lettuce plant a genotype associated with pest resistance, identify a lettuce plant with a genotype associated with pest resistance, and to select a lettuce plant with a genotype associated with pest resistance. In certain embodiments of methods of the invention, polymorphic nucleic acids can be used to produce a lettuce plant that comprises in its genome an introgressed locus associated with pest resistance. In certain embodiments of the invention, polymorphic nucleic acids can be used to breed progeny lettuce plants comprising a locus or loci associated with pest resistance.

Genetic markers may include "dominant" or "codominant" markers. "Codominant" markers reveal the presence of two or more loci (two per diploid individual). "Dominant" markers reveal the presence of only a single locus. Markers are preferably inherited in codominant fashion so that the presence of both loci at a diploid locus, or multiple loci in triploid or tetraploid loci, are readily detectable, and they are free of environmental variation, *i.e.,* their heritability is 1. A marker genotype typically comprises two marker loci at each locus in a diploid organism. The marker allelic composition of each locus can be either homozygous or heterozygous. Homozygosity is a condition where both loci at a locus are characterized by the same nucleotide sequence. Heterozygosity refers to a condition where the two loci at a locus are different.

Nucleic acid-based analyses for determining the presence or absence of the genetic polymorphism (*i.e.* for genotyping) can be used in breeding programs for identification, selection, introgression, and the like. A wide variety of genetic markers for the analysis of genetic polymorphisms are available and known to those of skill in the art. The analysis may be used to select for genes, portions of genes, QTL, loci, or genomic regions that comprise or are linked to a genetic marker that is linked to or associated with pest resistance in lettuce plants.

As used herein, nucleic acid analysis methods include, but are not limited to, PCR-based detection methods (for example, TaqMan assays), microarray methods, mass spectrometry-based methods and/or nucleic acid sequencing methods, including whole genome sequencing. In certain embodiments, the detection of polymorphic sites in a sample of DNA, RNA, or cDNA may be facilitated through the use of nucleic acid amplification methods. Such methods specifically increase the concentration of polynucleotides that span the polymorphic site, or include that site and sequences located either distal or proximal to it. Such amplified molecules can be readily detected by gel electrophoresis, fluorescence detection methods, or other means.

One method of achieving such amplification employs the polymerase chain reaction (PCR) (Mullis et al. (1986) Cold Spring Harbor Symp. Quant. Biol. 51:263-273; European Patent 50,424; European Patent 84,796; European Patent 258,017; European Patent 237,362; European Patent 201,184; U. S. Patent 4,683,202; U. S. Patent 4,582,788; and U. S. Patent 4,683,194), using primer pairs that are capable of hybridizing to the proximal sequences that define a polymorphism in its double-stranded form. Methods for typing DNA based on mass spectrometry can also be used. Such methods are disclosed in U.S. Patents Nos. 6,613,509 and 6,503,710, and references found therein.

Polymorphisms in DNA sequences can be detected or typed by a variety of effective methods well known in the art including, but not limited to, those disclosed in U.S. Patent Nos. 5,468,613, 5,217,863; 5,210,015; 5,876,930; 6,030,787; 6,004,744; 6,013,431; 5,595,890; 5,762,876; 5,945,283; 5,468,613; 6,090,558; 5,800,944; 5,616,464; 7,312,039; 7,238,476; 7,297,485; 7,282,355; 7,270,981 and 7,250,252, all of which are incorporated herein by reference in their entirety. However, the compositions and methods of the present invention can be used in conjunction with any polymorphism typing method to detect polymorphisms in genomic DNA samples. These genomic DNA samples used include but are not limited to, genomic DNA isolated directly from a plant, cloned genomic DNA, or amplified genomic DNA.

For instance, polymorphisms in DNA sequences can be detected by hybridization to locus-specific oligonucleotide (ASO) probes as disclosed in U.S. Patent Nos. 5,468,613 and 5,217,863. U.S. Patent No. 5,468,613 discloses locus specific oligonucleotide hybridizations where single or multiple nucleotide variations in nucleic acid sequence can be detected in nucleic acids by a process in which the sequence containing the nucleotide variation is amplified, spotted on a membrane and treated with a labeled sequence-specific oligonucleotide probe.

Target nucleic acid sequence can also be detected by probe ligation methods, for example as disclosed in U.S. Patent No. 5,800,944 where sequence of interest is amplified and hybridized to probes followed by ligation to detect a labeled part of the probe.

Microarrays can also be used for polymorphism detection, wherein oligonucleotide probe sets are assembled in an overlapping fashion to represent a single sequence such that a difference in the target sequence at one point would result in partial probe hybridization (Borevitz et al., Genome Res. 13:513-523 (2003); Cui et al., Bioinformatics 21:3852-3858 (2005). On any one microarray, it is expected there will be a plurality of target sequences, which may represent genes and/or noncoding regions wherein each target sequence is represented by a series of overlapping oligonucleotides, rather than by a single probe. This platform provides for high throughput screening of a plurality of polymorphisms. Typing of target sequences by microarray-based methods is described in U.S. Patent Nos. 6,799,122; 6,913,879; and 6,996,476.

Other methods for detecting SNPs and Indels include single base extension (SBE) methods. Examples of SBE methods include, but are not limited, to those disclosed in U.S. Patent Nos. 6,004,744; 6,013,431; 5,595,890; 5,762,876; and 5,945,283.

In another method for detecting polymorphisms, SNPs and Indels can be detected by methods disclosed in U.S. Patent Nos. 5,210,015; 5,876,930; and 6,030,787 in which an oligonucleotide probe having a 5' fluorescent reporter dye and a 3' quencher dye covalently linked to the 5' and 3' ends of the probe. When the probe is intact, the proximity of the reporter dye to the quencher dye results in the suppression of the reporter dye fluorescence, *e.g*. by Forster-type energy transfer. During PCR, forward and reverse primers hybridize to a specific sequence of the target DNA flanking a polymorphism while the hybridization probe hybridizes to polymorphism-containing sequence within the amplified PCR product. In the subsequent PCR cycle, DNA polymerase with 5' → 3' exonuclease activity cleaves the probe and separates the reporter dye from the quencher dye resulting in increased fluorescence of the reporter.

In another embodiment, a locus or loci of interest can be directly sequenced using nucleic acid sequencing technologies. Methods for nucleic acid sequencing are known in the art and include technologies provided by 454 Life Sciences (Branford, CT), Agencourt Bioscience (Beverly, MA), Applied Biosystems (Foster City, CA), LI-COR Biosciences (Lincoln, NE), NimbleGen Systems (Madison, WI), Illumina (San Diego, CA), and VisiGen Biotechnologies (Houston, TX). Such nucleic acid sequencing technologies comprise formats such as parallel bead arrays, sequencing by ligation, capillary electrophoresis, electronic microchips, "biochips," microarrays, parallel microchips, and single-molecule arrays.

Various genetic engineering technologies have been developed and may be used by those of skill in the art to introduce traits in plants. In certain aspects of the claimed invention, traits are introduced into lettuce plants via altering or introducing a single genetic locus or transgene into the genome of a variety or progenitor thereof. Methods of genetic engineering to modify, delete, or insert genes and polynucleotides into the genomic DNA of plants are well-known in the art.

In specific embodiments of the invention, improved lettuce lines can be created through the site-specific modification of a plant genome. Methods of genetic engineering include, for example, utilizing sequence-specific nucleases such as zinc-finger nucleases *(see,* for example, U.S. Pat. Appl. Pub. No. 2011/0203012); engineered or native meganucleases; TALE-endonucleases *(see,* for example, U.S. Pat. Nos. 8,586,363 and 9,181,535); and RNA-guided endonucleases, such as those of the CRISPR/Cas systems *(see,* for example, U.S. Pat. Nos. 8,697,359 and 8,771,945 and U.S. Pat. Appl. Pub. No. 2014/0068797). One embodiment of the invention thus relates to utilizing a nuclease or any associated protein to carry out genome modification. This nuclease could be provided heterologously within donor template DNA for templated-genomic editing or in a separate molecule or vector. A recombinant DNA construct may also comprise a sequence encoding one or more guide RNAs to direct the nuclease to the site within the plant genome to be modified. Further methods for altering or introducing a single genetic locus include, for example, utilizing single-stranded oligonucleotides to introduce base pair modifications in a plant genome *(see,* for example Sauer et al., Plant Physiol, 170(4):1917-1928, 2016).

Methods for site-directed alteration or introduction of a single genetic locus are well-known in the art and include those that utilize sequence-specific nucleases, such as the aforementioned, or complexes of proteins and guide-RNA that cut genomic DNA to produce a double-strand break (DSB) or nick at a genetic locus. As is well-understood in the art, during the process of repairing the DSB or nick introduced by the nuclease enzyme, a donor template, transgene, or expression cassette polynucleotide may become integrated into the genome at the site of the DSB or nick. The presence of homology arms in the DNA to be integrated may promote the adoption and targeting of the insertion sequence into the plant genome during the repair process through homologous recombination or non-homologous end joining (NHEJ).

In another embodiment of the invention, genetic transformation may be used to insert a selected transgene into a plant of the invention or may, alternatively, be used for the preparation of transgenes which can be introduced by backcrossing. Methods for the transformation of plants that are well-known to those of skill in the art and applicable to many crop species include, but are not limited to, electroporation, microprojectile bombardment, *Agrobacterium-*mediated transformation, and direct DNA uptake by protoplasts.

To effect transformation by electroporation, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wound tissues in a controlled manner.

An efficient method for delivering transforming DNA segments to plant cells is microprojectile bombardment. In this method, particles are coated with nucleic acids and delivered into cells by a propelling force. Exemplary particles include those comprised of tungsten, platinum, and preferably, gold. For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

An illustrative embodiment of a method for delivering DNA into plant cells by acceleration is the Biolistics Particle Delivery System, which can be used to propel particles coated with DNA or cells through a screen, such as a stainless steel or Nytex screen, onto a surface covered with target cells. The screen disperses the particles so that they are not delivered to the recipient cells in large aggregates. Microprojectile bombardment techniques are widely applicable and may be used to transform virtually any plant species.

*Agrobacterium-*mediated transfer is another widely applicable system for introducing gene loci into plant cells. An advantage of the technique is that DNA can be introduced into whole plant tissues, thereby bypassing the need for regeneration of an intact plant from a protoplast. Modern *Agrobacterium* transformation vectors are capable of replication in *E. coli* as well as *Agrobacterium,* allowing for convenient manipulations (Klee et al., Nat. Biotechnol., 3(7):637-642, 1985). Moreover, recent technological advances in vectors for *Agrobacterium-*mediated gene transfer have improved the arrangement of genes and restriction sites in the vectors to facilitate the construction of vectors capable of expressing various polypeptide coding genes. The vectors described have convenient multi-linker regions flanked by a promoter and a polyadenylation site for direct expression of inserted polypeptide coding genes. Additionally, *Agrobacterium* containing both armed and disarmed Ti genes can be used for transformation.

In those plant strains where *Agrobacterium-*mediated transformation is efficient, it is the method of choice because of the facile and defined nature of the gene locus transfer. The use of *Agrobacterium-*mediated plant integrating vectors to introduce DNA into plant cells is well known in the art (Fraley et al., Nat. Biotechnol., 3:629-635, 1985; U.S. Patent No. 5,563,055).

Transformation of plant protoplasts also can be achieved using methods based on calcium phosphate precipitation, polyethylene glycol treatment, electroporation, and combinations of these treatments *(see,* for example, Potrykus et al., Mol. Gen. Genet., 199:183-188, 1985; Omirulleh et al., Plant Mol. Biol., 21(3):415-428, 1993; Fromm et al., Nature, 312:791-793, 1986; Uchimiya et al., Mol. Gen. Genet., 204:204, 1986; Marcotte et al., Nature, 335:454, 1988). Transformation of plants and expression of foreign genetic elements is exemplified in Choi et al. (Plant Cell Rep., 13:344-348, 1994), and Ellul et al. (Theor. Appl. Genet., 107:462-469, 2003).

### V. Definitions

The following definitions are provided to better define the present invention and to guide those of ordinary skill in the art in the practice of the present invention. Unless otherwise noted, terms are to be understood according to conventional usage by those of ordinary skill in the relevant art.

As used herein, the term "plant" includes plant cells, plant protoplasts, plant cells of tissue culture from which lettuce plants can be regenerated, plant calli, plant clumps and plant cells that are intact in plants or parts of plants such as pollen, flowers, seeds, leaves, stems, or any portion thereof, or a non-regenerable portion of a plant part, including a cell, for example. As used in this context, a "non-regenerable" portion of a plant part is a portion that cannot be induced to form a whole plant or that cannot be induced to form a whole plant that is capable of sexual and/or asexual reproduction. In certain non-limiting embodiments, a non-regenerable portion of a plant or part thereof is a seed, leaf, flower, stem, root or cell, or any portion thereof.

As used herein, the term "population" means a genetically heterogeneous collection of plants that share a common parental derivation.

As used herein, the terms "variety" and "cultivar" mean a group of similar plants that by their genetic pedigrees and performance can be identified from other varieties within the same species.

As used herein, an "allele" refers to one of two or more alternative forms of a genomic sequence at a given locus on a chromosome.

A "quantitative trait locus" (QTL) is a chromosomal location that encodes for at least a first locus that affects the expressivity of a phenotype.

As used herein, a "marker" means a detectable characteristic that can be used to discriminate between organisms. Examples of such characteristics include, but are not limited to, genetic markers, biochemical markers, metabolites, morphological characteristics, and agronomic characteristics.

As used herein, the term "phenotype" means the detectable characteristics of a cell or organism that can be influenced by gene expression.

As used herein, the term "genotype" means the specific allelic makeup of a plant.

As used herein, "elite" or "cultivated" variety means any variety that has resulted from breeding and selection for superior agronomic performance. An "elite plant" refers to a plant belonging to an elite variety. Numerous elite varieties are available and known to those of skill in the art of lettuce breeding. An "elite population" is an assortment of elite individuals or varieties that can be used to represent the state of the art in terms of agronomically superior genotypes of a given crop species, such as lettuce. Similarly, an "elite germplasm" or elite strain of germplasm is an agronomically superior germplasm. The plants of the present invention are preferably elite plants.

As used herein, the term "introgressed," when used in reference to a genetic locus, refers to a genetic locus that has been introduced into a new genetic background, such as through backcrossing. Introgression of a genetic locus can be achieved through plant breeding methods and/or by molecular genetic methods. Such molecular genetic methods include, but are not limited to, various plant transformation techniques and/or methods that provide for homologous recombination, non-homologous recombination, site-specific recombination, and/or genomic modifications that provide for locus substitution or locus conversion.

As used herein, the terms "recombinant" or "recombined" in the context of a chromosomal segment refer to recombinant DNA sequences comprising one or more genetic loci in a configuration in which they are not found in nature, for example as a result of a recombination event between homologous chromosomes during meiosis.

As used herein, the term "linked," when used in the context of nucleic acid markers and/or genomic regions, means that the markers and/or genomic regions are located on the same linkage group or chromosome such that they tend to segregate together at meiosis.

As used herein, "tolerance locus" means a locus associated with tolerance or resistance to disease or pest. For instance, a tolerance locus according to the present invention may, in one embodiment, control tolerance or susceptibility to downy mildew disease.

As used herein, "tolerance" or "improved tolerance" in a plant refers to the ability of the plant to perform well, for example by maintaining yield, under disease conditions or upon pest infestations. Tolerance may also refer to the ability of a plant to maintain a plant vigor phenotype under disease conditions or under pest infestations. Tolerance is a relative term, indicating that a "tolerant" plant is more able to maintain performance compared to a different (less tolerant) plant (e. g. a different plant variety) grown in similar disease conditions or under similar pest pressure. One of skill will appreciate that plant tolerance to disease or pest conditions varies widely and can represent a spectrum of more-tolerant or less-tolerant phenotypes. However, by simple observation, one of skill can generally determine the relative tolerance of different plants, plant varieties, or plant families under disease or pest conditions, and furthermore, will also recognize the phenotypic gradations of "tolerance."

As used herein "resistance" or "improved resistance" in a plant to disease or pest conditions is an indication that the plant is more able to reduce disease or pest burden than a nonresistant or less resistant plant. Resistance is a relative term, indicating that a "resistant" plant is more able to reduce disease burden or pest burden compared to a different (less resistant) plant (e. g., a different plant variety) grown in similar disease conditions or pest pressure. One of skill will appreciate that plant resistance to disease conditions or pest infestation varies widely and can represent a spectrum of more-resistant or less-resistant phenotypes. However, by simple observation, one of skill can generally determine the relative resistance of different plants, plant varieties, or plant families under disease conditions or pest pressure, and furthermore, will also recognize the phenotypic gradations of "resistant."

As used herein, "resistance allele" means the nucleic acid sequence associated with tolerance or resistance to pest infestation.

"Sequence identity" and "sequence similarity" can be determined by alignment of two nucleotide sequences using global or local alignment algorithms. Sequences may then be referred to as "substantially identical" or "essentially similar" when they are optimally aligned by for example the programs GAP or BESTFIT or the Emboss program "Needle" (using default parameters) share at least a certain minimal percentage of sequence identity. These programs use the Needleman and Wunsch global alignment algorithm to align two sequences over their entire length, maximizing the number of matches and minimizing the number of gaps. Generally, the default parameters are used, with a gap creation penalty = 10 and gap extension penalty = 0. 5 (both for nucleotide and protein alignments). For nucleotides the default scoring matrix used is DNAFULL (Henikoff & Henikoff, PNAS 89:10915-10919; 1992). Sequence alignments and scores for percentage sequence identity may for example be determined using computer programs, such as EMBOSS as available on the world wide web under ebi.ac.uk/Tools/psa/emboss_needle. Alternatively, sequence similarity or identity may be determined by searching against databases such as FASTA, BLAST, etc., but hits should be retrieved and aligned pairwise to compare sequence identity. Two nucleic acid sequences have "substantial sequence identity" if the percentage sequence identity is at least 85%, 90%, 95%, 98%, 99% or more (*e.g.* at least 99.1, 99.2, 99.3, 99.4, 99.5, 99.6, 99.7, 99.8, 99.9 (as determined by Emboss "needle" using default parameters, *i.e.* gap creation penalty = 10, gap extension penalty = 0.5, using scoring matrix DNAFULL for nucleic acids)). Markers may sometimes exhibit variation, particularly in regions which are not recognized by the probes.

The term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and to "and/or. " When used in conjunction with the word "comprising" or other open language in the claims, the words "a" and "an" denote "one or more," unless specifically noted. The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps. Similarly, any plant that "comprises," "has" or "includes" one or more traits is not limited to possessing only those one or more traits and covers other unlisted traits.

### VI. Deposit Information

A deposit was made of at least 625 seeds of each of lettuce line BAG-LZ20-0001, lettuce line ZZL-LZ19-0002, lettuce line ZZL-LZ20-0002, and lettuce line ZZL-LZ21-0001. The deposits were made with the Provasoli-Guillard National Center for Marine Algae and Microbiota (NCMA), 60 Bigelow Drive, East Boothbay, Maine, 04544, USA. The deposits are assigned NCMA Accession Nos. 202110051, 202110049, 202110050, and 202110052, respectively, and the date of deposit was October 15, 2021. Access to the deposits will be available during the pendency of the application to persons entitled thereto upon request. The deposits have been accepted under the Budapest Treaty and will be maintained in the NCMA Depository, which is a public depository, for a period of 30 years, or 5 years after the most recent request, or for the enforceable life of the patent, whichever is longer, and will be replaced if nonviable during that period. Applicant does not waive any infringement of their rights granted under this patent or any other form of variety protection, including the Plant Variety Protection Act (7 U.S.C. 2321 et seq.).

### EXAMPLES

### Example 1. Testing for downy mildew resistance in lettuce

Pathology tests for determining resistance to downy mildew can be carried out using differential sets of lettuce varieties provided by the International Bremia Evaluation Board

(IBEB) that define resistance to different *B. lactucae* races. A lettuce variety that is commonly used as a susceptible control is the variety 'Green Towers,' however any other susceptible varieties may be used as controls in a pathology test. The resistant control used in the test depends on the *B. lactucae* isolate that is being used, but the differential set generally offers multiple resistant variety options per downy mildew isolate. The IBEB initiative provides contacts where validated seeds of the varieties of the differential set can be obtained as well as validated samples of *B. lactucae* isolates.

A pathology test ideally evaluates between 15-30 plants per variety. The experiment can be performed with plants grown in soil or on artificial substrate containing plant growth medium, *e.g.* 0.5x Hoagland solution. The plants are ideally germinated and grown at a temperature of 15°C with 12 hours of light. After about 7 days, when the cotyledons have fully opened, the plants are inoculated with *B. lactucae* conidia by spraying them with inoculum suspension. The inoculum suspension is produced by washing leaves containing a sporulating *B. lactucae* infection, which is about 7-10 days after inoculation, with sterile water, sieved to remove remaining plant parts, and diluted with sterile water to a final concentration of 1×10⁴ conidia/ml.

After inoculation, the plants are maintained in an environment with very high humidity (around 100%) at 15°C with 12-16 hours of light. Seven to 10 days post inoculation, the first sporulation should be visible. The first reading in the experiment should be done 10 days post-inoculation. A second reading should be performed 14 days post-inoculation. A 1-9 rating scale can be used to score the level of resistance observed in the experiment, where a score of 1 indicates no infection and a score of 9 indicates heavy sporulation. Intermediate scores are as follows: 2 indicates observable necrosis only; 3 indicates observable necrosis and mild sporulation; and 7 indicates moderate sporulation. A similar scoring scale has been developed by and is available from the IBEB. The experiment is considered successful when the susceptible control is sporulating and the other controls score as expected.

### Example 2. Fine mapping of resistance loci

The *B. lactucae* resistance gene cluster on *L*. *sativa* chromosome 2 spans a 9 cM region residing at the beginning of the chromosome while the *B. lactucae* resistance gene cluster on *L*. *sativa* chromosome 4 spans a 6.5 cM region at the end of the chromosome. These gene clusters are treated by breeders as allelic because the individual genes are so closely linked that it is extremely difficult to combine alleles from different sources within the gene clusters. Although recombination within a gene cluster has rarely occurred, the resulting recombinant genomic segments were shown to provide an incomplete resistance profile (WO 2013/124210). In view of this, once a resistance locus has been sorted into one of the known gene clusters, mapping efforts are typically stopped because that resistance locus is considered to be allelic with other loci within the gene cluster.

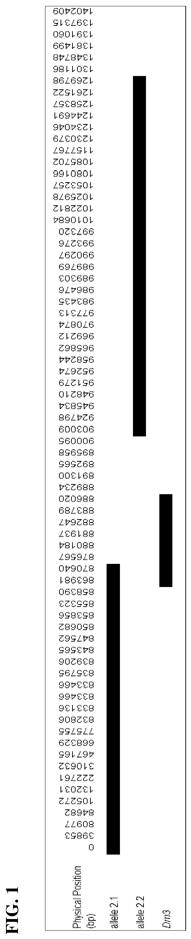

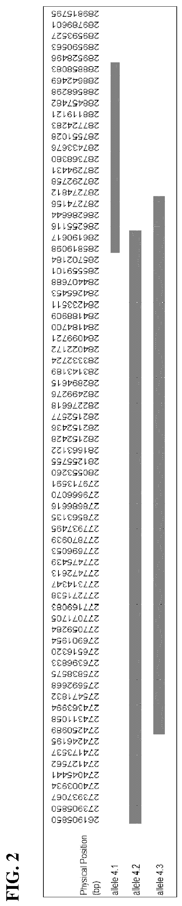

In order to predictively combine resistance loci located in the same gene cluster, a haplotype analysis approach was taken to fine map the different resistance loci within each gene cluster. In this approach, the fingerprint of related lines with and without the resistance allele were compared to a reference, *e.g.* the resistance donor. The genomic sequence in the resistance interval was broken up into small genomic intervals and for each interval a haplotype was determined for each line. These haplotypes were then compared to determine the genomic region where the resistance alleles are located. Through this method, it was found that for the loci on chromosome 2, most of the alleles were mapped to overlapping regions in the gene cluster. However, resistance allele 2.2 was found to be in close proximity but not entirely overlapping with the *Dm3* gene and allele 2.1, although the genetic distance was found to be between 0-0.5 cM, making the possibility of recombination between the two loci unlikely (FIG. 1). The situation for the gene cluster on chromosome 4 was found to be similar, and most resistance alleles were found to occupy overlapping regions in the gene cluster (FIG. 2). Only allele 4.1 and allele 4.2 did not map entirely to overlapping regions, even though recombination was unlikely due to a mapped genetic distance of 0.1 cM between the two alleles.

### Example 3. Development of novel alleles conferring

### broad-spectrum resistance to B. lactucae

Novel *B. lactucae* coupling events were generated through recombination by combining allele 2.1 and allele 2.2 in cis-linkage on chromosome 2 and by combining allele 2.2 with the *Dm3* gene in cis-linkage on chromosome 2.

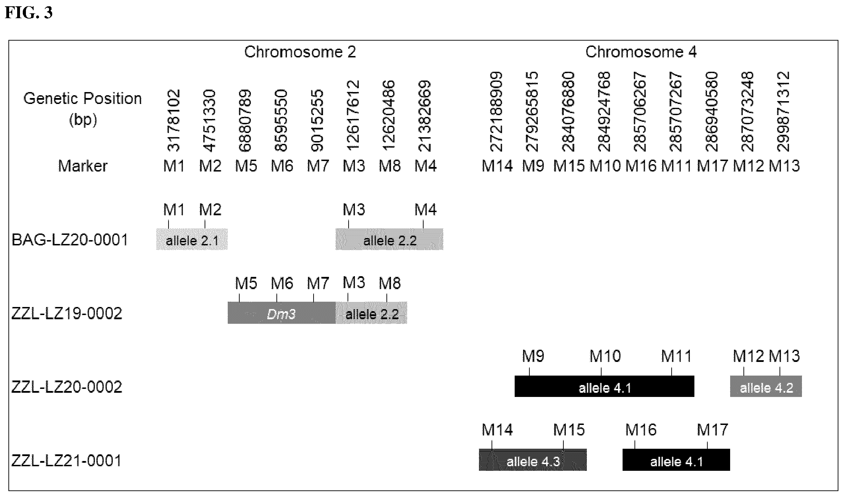

Lettuce line BAG-LZ20-0001 comprises a novel *B. lactucae* coupling event that combines the resistance allele 2.1 and allele 2.2 in a cis-configuration on chromosome 2. Marker locus M2 (SEQ ID NO: 6) was developed to track allele 2.1 and marker locus M3 (SEQ ID NO: 11) was developed to track allele 2.2. This novel coupling event of allele 2.1 and allele 2.2 is located between marker loci M1 (SEQ ID NO: 1) and M4 (SEQ ID NO: 16) on chromosome 2. Marker loci M2 and M3 can be used to select the resistance allele from BAG-LZ20-0001 on chromosome 2 (FIG. 3). This new resistance allele comprising the coupling event was tested against all commercially relevant *B. lactucae* isolates. It was found that the complete resistance profiles of each of the two individual alleles had been preserved and the coupling event combined the resistance profiles. The novel *B. lactucae* resistance allele from line BAG-LZ20-0001 confers resistance to European *B. lactucae* races B1:16-B1:36 and U.S. *B. lactucae* races CA:5-CA:9.

Lettuce line ZZL-LZ19-0002 comprises a novel *B. lactucae* resistance allele that combines the resistance gene *Dm3* and allele 2.2 in a cis-configuration on chromosome 2. Marker locus M3 (SEQ ID NO: 11) was developed to track allele 2.2 and marker loci M5 (SEQ ID NO: 21), M6 (SEQ ID NO: 26), and M7 (SEQ ID NO: 31) were developed to track the *Dm3* gene. This novel coupling event of the *Dm3* gene and allele 2.2 is located between marker loci M5 (SEQ ID NO: 21) and M8 (SEQ ID NO: 36) on chromosome 2 (FIG. 3). This new resistance allele comprising the coupling event was tested against all commercially relevant *B. lactucae* isolates. It was found that the complete resistance profiles of each of the two individual alleles had been preserved and the coupling event combined the resistance profiles. The novel *B*. *lactucae* resistance allele from line ZZL-LZ19-0002 confers resistance to European *B. lactucae* races B1:16-B1:31 and B1:33-B1:36 and U.S. *B. lactucae* races CA:5-CA:9. Line ZZL-LZ19-0002 also comprises a *B. lactucae* resistance allele on chromosome 4 that confers the plants of this line resistance to European *B. lactucae* isolate Bl:32. The markers that can be used to track the individual resistance alleles and the novel coupling events on chromosome 2 are shown in Table 1 below.

**Table 1. Markers to track B. lactucae resistance allele on chromosome 2.**

| **Marker** | **Chr** | **Favorable Allele** | **SNP change** | **SNP position in marker (bp)** | **SNP Position in Public Genome (bp)** | **Marker Sequence (SEQ ID NO)** | **Fwd Primer (SEQ ID NO)** | **Rev Primer (SEQ ID NO)** | **Probe 1 (SEQ ID NO)** | **Probe 2 (SEQ ID NO)** |
|---|---|---|---|---|---|---|---|---|---|---|
| M1 | 2 | C | C/T | 96 | 3,178,102 | 1 | 2 | 3 | 4 | 5 |
| M2 | 2 | A | A/T | 44 | 4,751,330 | 6 | 7 | 8 | 9 | 10 |
| M3 | 2 | T | C/T | 58 | 12,617,612 | 11 | 12 | 13 | 14 | 15 |
| M4 | 2 | T | C/T | 57 | 21,382,669 | 16 | 17 | 18 | 19 | 20 |
| M5 | 2 | T | A/T | 101 | 6,880,789 | 21 | 22 | 23 | 24 | 25 |
| M6 | 2 | G | A/G | 101 | 8,595,550 | 26 | 27 | 28 | 29 | 30 |
| M7 | 2 | C | C/T | 101 | 9,015,255 | 31 | 32 | 33 | 34 | 35 |
| M8 | 2 | C | T/C | 61 | 12,620,486 | 36 | 37 | 38 | 39 | 40 |

Novel *B. lactucae* recombination events were also generated through recombination by combining allele 4.1 and allele 4.2 in cis-linkage on chromosome 4 and by combining allele 4.1 and allele 4.3 in cis-linkage on chromosome 4.

Lettuce line ZZL-LZ20-0002 comprises a novel *B. lactucae* coupling event that combines the resistance allele 4.1 and resistance allele 4.2 in a cis-configuration on chromosome 4. This novel coupling event of allele 4.1 and allele 4.2 is located between marker loci M9 (SEQ ID NO: 41) and M13 (SEQ ID NO: 61) on chromosome 4. In addition, marker loci M10 (SEQ ID NO: 46), M12 (SEQ ID NO: 56), and M13 (SEQ ID NO: 61) can be used to select for the novel resistance allele containing the coupling event on chromosome 4 (FIG. 3). The novel *B. lactucae* resistance allele from line ZZL-LZ20-0002 confers resistance to the European *B. lactucae* races B1:16-B1:36 and U.S. *B. lactucae* races CA:5-CA:9.

Lettuce line ZZL-LZ21-0001 comprises a novel *B. lactucae* resistance allele that combines the resistance allele 4.3 and resistance allele 4.1 in a cis-configuration on chromosome 4. This novel coupling event of allele 4.3 and allele 4.1 is located between marker loci M14 (SEQ ID NO: 61) and M17 (SEQ ID NO: 81) on chromosome 4. In addition, marker loci M15 (SEQ ID NO: 71) and M16 (SEQ ID NO: 76) can be used to select for the novel resistance allele containing the coupling event on chromosome 4 (FIG. 3). The novel *B. lactucae* resistance allele from line ZZL-LZ21-0001 confers resistance to the European *B. lactucae* races B1:16-B1:36 and U.S. *B. lactucae* races CA:6-CA:9. The markers that can be used to track the novel alleles comprising a coupling event on chromosome 4 are shown in Table 2 below.

**Table 2. Markers to track B. lactucae resistance allele on chromosome 4.**

| **Marker** | **Chr** | **Favorable Allele** | **SNP change** | **SNP position in marker (bp)** | **SNP Position in Public Genome (bp)** | **Marker Sequence (SEQ ID NO)** | **Fwd Primer (SEQ ID NO)** | **Rev Primer (SEQ ID NO)** | **Probe 1 (SEQ ID NO)** | **Probe 2 (SEQ ID NO)** |
|---|---|---|---|---|---|---|---|---|---|---|
| M9 | 4 | A | A/T | 79 | 279,265,815 | 41 | 42 | 43 | 44 | 45 |
| M10 | 4 | A | A/T | 101 | 284,924,768 | 46 | 47 | 48 | 49 | 50 |
| M11 | 4 | C | C/T | 87 | 285,707,267 | 51 | 52 | 53 | 54 | 55 |
| M12 | 4 | G | G/A | 101 | 287,073,248 | 56 | 57 | 58 | 59 | 60 |
| M13 | 4 | T | C/T | 497 | 299,871,312 | 61 | 62 | 63 | 64 | 65 |
| M14 | 4 | C | T/C | 61 | 272,188,909 | 66 | 67 | 68 | 69 | 70 |
| M15 | 4 | G | A/G | 101 | 284,076,880 | 71 | 72 | 73 | 74 | 75 |
| M16 | 4 | C | T/C | 101 | 285,706,267 | 76 | 77 | 78 | 79 | 80 |
| M17 | 4 | C | C/T | 101 | 286,940,580 | 81 | 82 | 83 | 84 | 85 |

## Claims

1. A *Lactuca sativa* plant comprising a recombinant chromosomal segment that comprises a first allele that confers resistance to *Bremia lactucae* and a second allele that confers resistance to *Bremia lactucae,* wherein said first allele and second allele are in *cis* configuration on chromosome 2, and wherein said first allele comprises allele 2.2 and wherein said second allele comprises allele 2.1 or *Dm3,* wherein
a) said recombinant chromosomal segment comprises a marker locus selected from the group consisting of marker locus M1 (SEQ ID NO: 1), marker locus M2 (SEQ ID NO: 6), marker locus M3 (SEQ ID NO: 11), marker locus M4 (SEQ ID NO: 17), marker locus M5 (SEQ ID NO: 21), marker locus M6 (SEQ ID NO: 26), marker locus M7 (SEQ ID NO: 31), and marker locus M8 (SEQ ID NO: 36) on chromosome 2;
b) the plant is homozygous for said recombinant chromosomal segment; or
c) a representative sample of seed comprising said recombinant chromosomal segment has been deposited under NCMA Accession No. 202110051 or NCMA Accession No. 202110049.

2. The plant of claim 1, wherein said recombinant chromosomal segment comprises marker locus M3 (SEQ ID NO: 11) and a marker locus selected from the group consisting of marker locus M2 (SEQ ID NO: 6), marker locus M5 (SEQ ID NO: 21), marker locus M6 (SEQ ID NO: 26), and marker locus M7 (SEQ ID NO: 31) on chromosome 2.

3. A plant part of the plant of claim 1, wherein said plant part comprises said recombinant chromosomal segment.

4. The plant part of claim 3, wherein said plant part is a cell, a seed, a root, a stem, a leaf, a head, a flower, or pollen.

5. A seed that produces the plant of claim 1.

6. A recombinant DNA segment comprising a first *Bremia lactucae* resistance allele and a second *Bremia lactucae* resistance allele, wherein said first allele and second allele are in *cis* configuration, and wherein said first allele comprises allele 2.2 and wherein said second allele comprises allele 2.1 or *Dm3* , wherein
a) said recombinant DNA segment comprises the sequence of marker locus M3 (SEQ ID NO: 11) and a sequence selected from the group consisting of marker locus M2 (SEQ ID NO: 6), marker locus M5 (SEQ ID NO: 21), and marker locus M6 (SEQ ID NO: 26);
b) the recombinant DNA segment is further defined as comprised within a plant, plant part, plant cell, or seed; or
c) a representative sample of seed comprising said DNA segment has been deposited under NCMA Accession No. 202110051 or NCMA Accession No. 202110049.

7. A *Lactuca sativa* plant comprising a recombinant chromosomal segment that comprises a first allele that confers resistance to *Bremia lactucae* and a second allele that confers resistance to *Bremia lactucae* wherein said first allele and second allele are in *cis* configuration on chromosome 4, and wherein said first allele comprises allele 4.1 and wherein said second allele comprises allele 4.2 or allele 4.3, wherein
a) said recombinant chromosomal segment comprises a marker selected from the group consisting of marker locus M9 (SEQ ID NO: 41), marker locus M10 (SEQ ID NO: 46), marker locus M11 (SEQ ID NO: 51), marker locus M12 (SEQ ID NO: 56), marker locus M13 (SEQ ID NO: 61), marker locus M14 (SEQ ID NO:66), marker locus M15 (SEQ ID NO: 71), marker locus M16 (SEQ ID NO: 76), and marker locus M17 (SEQ ID NO: 81) on chromosome 4;
b) the plant is homozygous for said recombinant chromosomal segment; or
c) a representative sample of seed comprising said recombinant chromosomal segment has been deposited under NCMA Accession No. 202110050 or NCMA Accession No. 202110052.

8. The plant of claim 7, wherein said recombinant chromosomal segment comprises marker locus selected from the group consisting of marker locus M9 (SEQ ID NO: 41), marker locus M10 (SEQ ID NO: 46), marker locus M11 (SEQ ID NO: 51), marker locus M16 (SEQ ID NO: 76), and marker locus M17 (SEQ ID NO: 81) and a marker locus selected from the group consisting of marker locus M12 (SEQ ID NO: 56), marker locus M13 (SEQ ID NO: 61, marker locus M14 (SEQ ID NO: 66), and marker locus M15 (SEQ ID NO: 71) on chromosome 4.

9. A plant part of the plant of claim 7, wherein said plant part comprises said recombinant chromosomal segment.

10. The plant part of claim 9, wherein said plant part is a cell, a seed, a root, a stem, a leaf, a head, a flower, or pollen.

11. A seed that produces the plant of claim 7.

12. A recombinant DNA segment comprising a first *Bremia lactucae* resistance allele and a second *Bremia lactucae* resistance allele, wherein said first allele and second allele are in *cis* configuration, and wherein said first allele comprises allele 4.1 and wherein said second allele comprises allele 4.2 or allele 4.3, wherein
a) said recombinant DNA segment comprises the sequence of marker locus M9 (SEQ ID NO: 41), marker locus M10 (SEQ ID NO: 46), marker locus M11 (SEQ ID NO: 51), marker locus M16 (SEQ ID NO: 76), and marker locus M17 (SEQ ID NO: 81) and a marker locus selected from the group consisting of marker locus M12 (SEQ ID NO: 56), marker locus M13 (SEQ ID NO: 61, marker locus M14 (SEQ ID NO: 66), and marker locus M15 (SEQ ID NO: 71);
b) the recombinant DNA segment is further defined as comprised within a plant, plant part, plant cell, or seed; or
c) a representative sample of seed comprising said DNA segment has been deposited under NCMA Accession No. 202110050 or NCMA Accession No. 202110052.

13. A method for selecting a *Lactuca sativa* plant with broad-spectrum resistance to *Bremia lactucae* comprising detecting in a plant a recombinant chromosomal segment comprising a first *Bremia lactucae* resistance allele and a second *Bremia lactucae* resistance allele within a recombinant chromosomal segment flanked in the genome of said plant, wherein the method comprises:
a) detecting the presence of marker locus M1 (SEQ ID NO: 1) and marker locus M4 (SEQ ID NO: 17) on chromosome 2; or
b) detecting the presence of marker locus M9 (SEQ ID NO: 41) and marker locus M17 (SEQ ID NO: 81) on chromosome 4,
wherein said first and second *Bremia lactucae* resistance alleles confer to said plant broad-spectrum resistance to *Bremia lactucae* relative to a plant lacking said alleles.

14. A tissue culture comprising the cell of claim 4.

15. Plant according to any one of claims 1 to 5 or 7 to 11, wherein the plant is an elite *Lactuca sativa* plant comprising a recombinant chromosomal segment that comprises a first allele that confers resistance to *Bremia lactucae* and a second allele that confers resistance to *Bremia lactucae.*
